# EUROPEAN PATENT APPLICATION

(11) **EP 0 921 132 A1**
(43) Date of publication of application: **09.06.1999**
(21) Application number: 97924310.2
(22) Date of filing: 04.06.1997
(51) Int. Cl.: C07K 14/705

(54) **NOVEL HUMAN RECEPTOR TYPE TYROSINE KINASE-LIKE PROTEIN**

(30) Priority: 04.06.1996 JP 14184996
(71) Applicant: SHIONOGI & CO., LTD., Osaka-shi, Osaka 541-0045 (JP)
(72) Inventor: MATSUI, Toshimitsu, Kobe-shi, Hyogo 651-13 (JP)
(74) Representative: VOSSIUS & PARTNER
(86) International application number: JP9701887
(87) International publication number: WO9746586

(57) **Abstract**

A receptor type tyrosine-kinase like protein having an Eph family structure and seeming showing signal modification and/or amplification.

## Description

### Technical Field

This invention relates to a novel receptor having a human receptor type tyrosine kinases like structure. More specifically, it relates to a receptor type tyrosine kinases like protein having Eph family structure.

### Background Art

An intercellular signal transmission system is proposed as a means of cells for receiving stimulation from surrounding environment. By the system, cells can communicate with each other to control behavior of each cell in a whole organism's behalf. Intercellular transmission is essential for exact regulation of various cell processes such as differentiation, proliferation, adhesion, metabolism and secretion. An example for the general principle of the signal transmission includes the following mechanism. Signal transmission molecules such as hormone, growth factor, neurotransmitter are secreted from signal-producing cells, and target cells receiving the signal respond to it through specific proteins designated receptors. Binding of these signal transmission molecules to receptors would be a trigger of reaction cascades to cause both of amplification of original stimulation and synchronous control of various cell processes.

One of principal features in the signal transmission process is reversible phosphorylation of some proteins. Phosphorylation or dephosphorylation of amino acid residues in a protein is a trigger of a conformation change of a protein to be regulated, and changes biological properties of the protein. Phosphorylation of proteins is caused by protein kinases. Protein kinases are classified by amino acid residues to which they react. One of the classes is a serine-threonine kinase to react with serine and threonine residues. Another class is a tyrosine kinase to react with a tyrosine residue. The protein phosphorylation at a tyrosine residue has been of interest since the discovery of many cancer gene products and growth factor receptors having an intrinsic tyrosine kinase activity. Nowadays, an importance of phosphorylation of a tyrosine residue in growth factor signal transmission, cell cycle progress and malignant transformation is fully recognized, and it is also recognized that an activation and overexpression of a tyrosine kinase results in destruction of normal growth control pathways and then in carcinogenesis (Hunter, 1991). Protein kinases including a tyrosine kinase can be defined as a receptor, that is, a transmembrane-type ligand-binding protein, and as a non-receptor.

As described above, the receptor-type tyrosine-kinase plays an important role in cellular proliferation and differentiation of a wide variety of cell types (Aaronson, 1991 ; Schlessinger & Ullrich, 1992). Many receptors which combine with epidermal growth factor(EGF), platelet-derived growth factor(PDGF), fibroblast growth factor(FGF), insulin and so on have been reported as the receptor type tyrosine kinases. The receptor type tyrosine kinases can be classified into some subfamilies based upon a homology of an extracellular region's domain construction and a cytoplasmic tyrosine kinases construction. Eph family is one of these subfamilies, characterized by one cysteine rich domain and two fibronectin type domain III on the extracellular region, and includes many receptors such as Eph, Eck, Elk,Cek5, Cek7 and so on (Tuzi & Gullick, 1994).

### Summary of Invention

Some of Eph family receptors (Elk, Mek4, Sek, Cek5, Ehk1, and Ehk2) are abundantly expressed in brain. Since it is suggested that these receptors take part in positional labeling that may guide the development of neural topographic maps. Many Eph family receptors are abundantly expressed in early embryos. Since it is also suggested that these receptors play an important role in development of early embryos. Eph family receptors are very interesting proteins. The inventors made an attempt to isolate a novel Eph family receptor using a probe corresponding to a kinase domain of Htk gene which is a member of Eph family receptors. As a result, the inventors found out a specific Eph family receptor gene expressed in human normal tissues. A protein encoded by this gene was designated HEP (Human Eph-like Protein) receptor. The HEP receptor is presumed to have unique functions because six of ten amino acid residues which are critical for kinase activity and invariant in all tyrosine kinases were substituted with other amino acid residues.

The present invention relates to Eph family receptor type tyrosine kinases like protein which has unique structure including the HEP receptor. In the preferred embodiments, an invariant lysine residue in an ATP-binding site and an invariant aspartic acid residue in an transphosphorylation site of Eph family receptor type tyrosine kinase were substituted with a glutamine residue and a serine residue, respectively, in the protein of this invention. In addition to the above two substitutions, an invariant glutamic acid residue in subdomain III and an invariant asparagin residue in kinase subdomain VI of Eph family receptor type tyrosine kinase may be substituted with an arginine and a serine, respectively, and a triplet Asp-Phe-Gly in kinase subdomain VII may be substituted with a triplet Arg-Leu-Gly. It is more preferred that the protein contains an amino acid sequence from 17th Leu to 1,006th Val of SEQ ID NO.1 in the sequence listing, which is the HEP receptor. The present invention includes a protein which contains the amino acid sequence from first Met to 1,006th Val including a signal sequence.

Additionally, the invention provides a DNA molecule which contains a nucleotide sequence encoding the protein of this invention. A preferred DNA molecule contains a nucleotide sequence from 799th A (adenine) to 3,816th C (cytosine) of SEQ ID No.2 in the sequence listing.

### Explanation of the terms

The term "invariant" prefixed to the above amino acid residues means constitutional amino acid residues which are critical for kinase activity common to various protein kinases and was first found out by Hanks et al.(1988) who made a common feature research by aligning amino acid sequences of 65 kinds of protein kinases. From the report of Hanks et al., there are 10 invariant amino acid residues, which can be shown as follows based upon the sequence of bovine adenosine 3',5'-monophosphate (cAMP)-dependant protein kinase catalytic subunit α (cAPK-α).
Gly⁵², Ala⁷⁰, Lys⁷², Glu⁹¹, Asp¹⁶⁶, Asn¹⁷¹, Asp¹⁸⁴, Gly¹⁸⁶, Glu²⁰⁸, and Arg²⁸⁰.

Hanks et al. also found out that protein kinases have repeating structure of a highly conserved sequence and a low conserved sequence in a cytoplasmic region. The term "kinase subdomain" means the highly conserved sequence. There are 11 kinase subdomains distinguished by being numbered in Roman numerals.

"ATP binding site" is a site associating with the binding to ATP and located in subdomain II. "Transphosphorylation site" is a site interacting with a phosphate group of ATP and located in subdomain VI.

### Disclosure of Invention

Explained in more detail below is the Eph family receptor type tyrosine kinase like protein of the present invention having unique structure, specifically HEP receptor.

### Isolation of a DNA molecule encoding the HEP receptor

A probe corresponding to a kinase domain of human Htk (Hepatoma transmembrane kinase) was prepared. Then, human brain and blood cell cDNA libraries were screened under the condition of weak stringency by the cross-hybridization method using the probe. Through this step, a DNA molecule encoding HEP receptor having unique structure was isolated. A PCR method is generally used to isolate homologous genes using a primer having a sequence common to kinase proteins, but it is disadvantageous to the cloning of DNA molecules encoding proteins substituted in consensus sequences like the protein of this invention.

### Primary structure of the HEP receptor

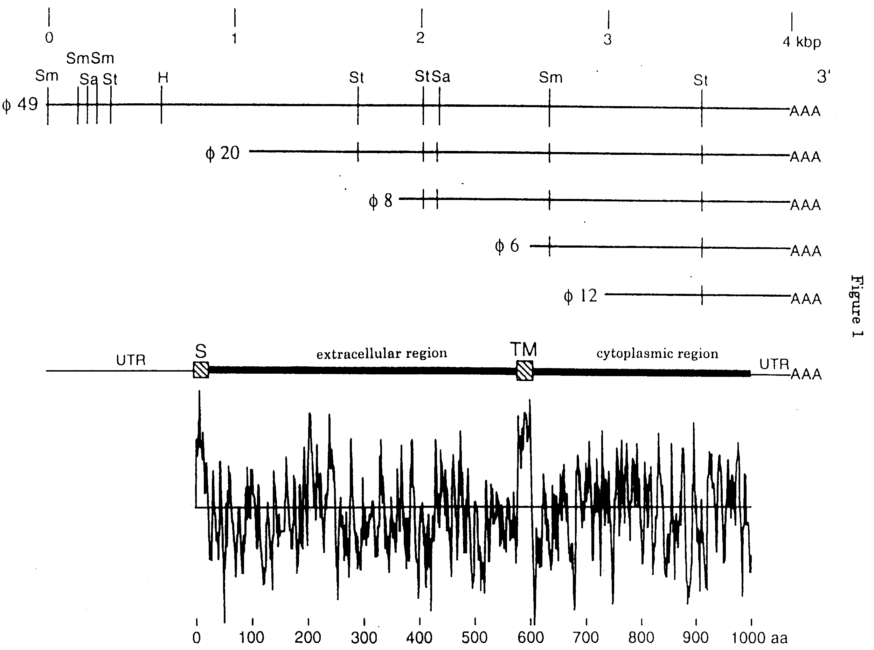

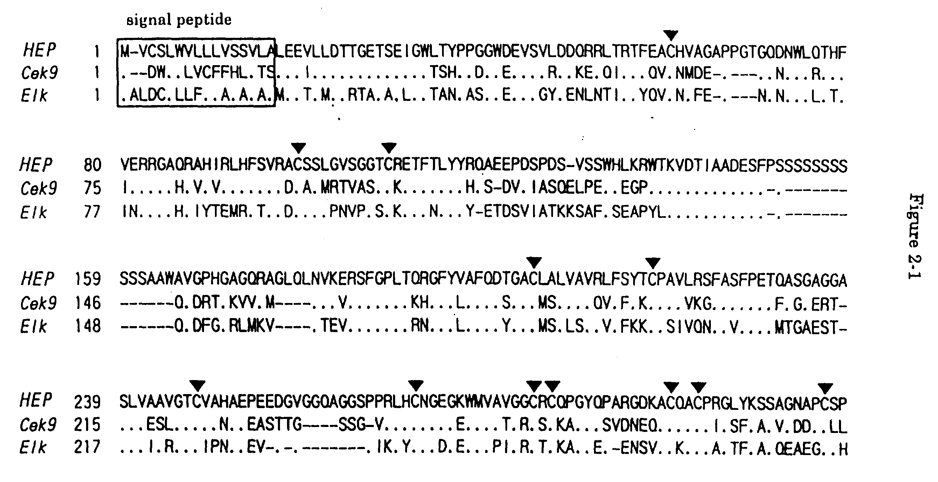

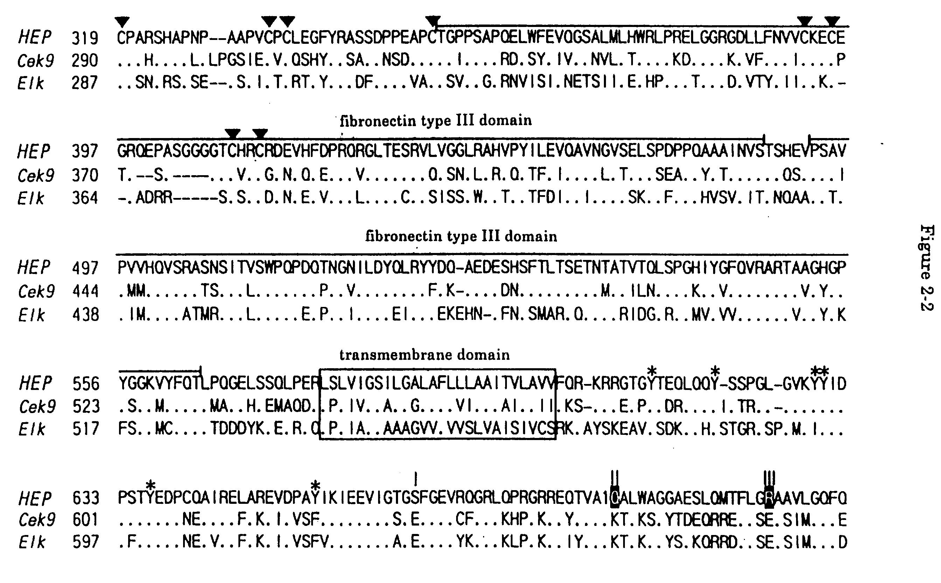

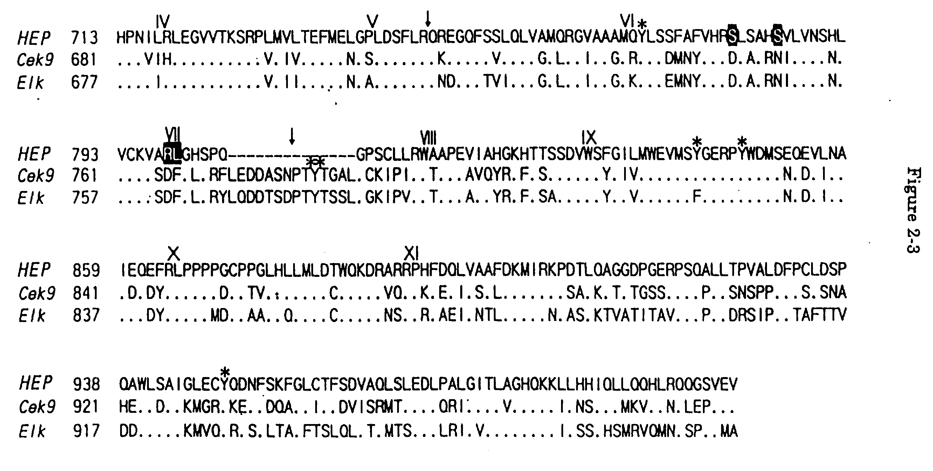

An amino acid sequence was deduced from the isolated DNA molecule encoding HEP receptor. First methionine residue (corresponding to ATG codon from 799th to 801st in SEQ ID No.2) was surrounded by a typical conserved sequence (Kozak, 1984) (Figure 2) and followed by a hydrophobic amino acid sequence characteristic of a signal peptide (Von Heijne, 1986). Another hydrophobic amino acid sequence (amino acids from 578th to 603rd) is seemed to function as a transmembrane domain. This domain (TM ) divides HEP receptor molecule into an extarcellular domain and a cytoplasmic domain (Figure 1).

Figure 1 shows a structure chart of HEP receptor cDNA clone and the result of hydrophobicity analysis (Kyte and Doolitte). Positive numerical value in hydrophobicity analysis means that the residue is hydrophobic. In the figure, S means a deduced signal pepetide, TM means a deduced transmembrane domain, UTR means a non-translation region, Sm means a SmaI restriction site, Sa means a SacI restriction site, St means a StuI restriction site, and H means a HidIII restriction site.

### Homology between known Eph family receptors and HEP receptor

Computer analysis showed that the deduced amino acid sequence of HEP receptor was highly homologous to those of Eph-family receptor tyrosine kinases. The highest homology was observed with chicken embryo kinase 9 (CEK9), a member of the avian Eph-family, followed by rat Eph like kinase (Elk) and human Eph related kinase (Erk) and avian homologue Cek5. The amino acid sequence of HEP receptor, in comparison with those of CEK9, ELK, ERK and CEK5, has overall identities of 59 %, 48 %, 47 % and 47 %, identities in the extracellular region of 57 %, 44 %, 44 % and 42 %, and identities in the cytoplasmic region of 60 %, 54 %, 50 % and 53 %, respectively.

Figure 2 is shown for help in understanding these homologies. Figure 2 shows the deduced amino acid sequence of HEP receptor and alignments to Cek9 and Elk. The putative signal peptide and transmembrane domain are boxed. Dots in the Cek9 and Elk sequences mean the same amino acid residue as that of HEP receptor, and hyphens mean absence of a residue. Conserved cysteine residues are marked by inverted triangles. Two fibronectin type III domains in the extracellular region are overlined. Kinase subdomains in the cytoplasmic region are numbered in Roman numerals from I to XI. The conserved tyrosine residues in the cytoplasmic region are shown with asterisks. The tyrosine residue corresponding to Tyr⁴¹⁶ for pp60^{c-src}, one of the principal autophosphorylation sites, is shown with double asterisks. Introns are located at the positions indicated by arrows. Amino acids shown in white mean the invariant amino acids which are substituted in HEP receptor but are invariant in all the other tyrosine kinases.

### Features of the HEP receptor sequence in extracellular region

The extracellular region of 577 amino acids shared several structural features with that of the Eph family, such as cystein-rich region containing 20 conserved cystein residues and two fibronectin type III domain (Figure 2). Therefore, the HEP receptor of the present invention was judged to be a Eph family receptor type.

### Features of the HEP receptor sequence in cytoplasmic region

### 1 ) Kinase subdomain

There are 11 kinase subdomains which were found out by Hanks et al.(1988) and conserved in the cytoplasmic region of every protein kinase. The HEP receptor has all of the 11 kinase subdomains in the cytoplasmic region.

### 2 ) Conserved tyrosine residues

The HEP receptor retains almost all conserved tyrosine residues which Eph family tyrosine kinase proteins should retain.

### 3 ) Features of other conserved sites

There is a conserved sequence, Gly-X-Gly-X-X-Gly, in kinase subdomain I, which is found in not only a protein kinase but also many nucleotide-binding proteins (Hanks, 1988). The HEP receptor contains Gly-Thr-Gly-Ser-Phe-Gly as this conserved sequence Gly-X-Gly-X-X-Gly at positions 662-667.

The HEP receptor has an invariant alanine at position 685 corresponding to an invariant alanine at position 70 shown in the figure of Hanks et al.(1988).

### 4 ) Substitution of invariant amino acids

Six of the amino acids invariant in all protein kinases are substituted in the kinase domain of the HEP receptor.

First, invariant lysine was substituted with glutamine in the amino acid sequence of HEP receptor (Figure 2, 687th of HEP receptor). The invariant lysine is within the ATP-binding site corresponding to Lys⁷² in bovine adenosine 3',5'-monophosphate (cAMP)-dependent protein kinase catalytic subunit α (cAPK-α) (Hanks et al., 1988). Secondly, invariant aspartic acid (corresponding to Asp¹⁶⁶ for cAPK-α) in the phosphoytansfer site of kinase subdomain VI, was substituted with serine at position 780, while its neighboring residues were well conserved. Thirdly, invariant glutamic acid (Glu⁹¹ for cAPK-α) in kinase subdomain III was replaced with arginine at position 704. Fourthly, invariant asparagine (Asn¹⁷¹ for cAPK-α) in kinase subdomain VI was changed into serine at position 785. Furthermore, the triplet corresponding to Asp¹⁸⁴, Phe¹⁸⁵ and Gly¹⁸⁶ for cAPK-α conserved in most of tyirosine kinases was altered to Arg⁷⁹⁸, Leu⁷⁹⁹ and Gly⁸⁰⁰ in the HEP receptor, of which two amino acids were replaced.

These six substitutions were found not only in the clones derived from both normal human brain and hematopoietic cell cDNA libraries, but also in clones derived from normal human pancreas cDNA. Individual origin of these libraries is different from each other. Furthermore, the substitutions were confirmed in the normal human genomic DNA clones obtained by PCR using the primers designed according to the HEP receptor cDNA sequence. These experimental results clearly show that the six substitutions described above are neither mutations caused by errors on translation and replication of the gene, nor artificial mutations caused by errors on preparing cDNA. They are intrinsic substitutions, not based upon gene polymorphism originating from individual difference.

### 5 ) Features of deleted parts

The HEP receptor lacked a highly conserved 14-amino acid stretch in subdomain VII, which includes a major autophosphorylation site corresponding to Tyr⁴¹⁶ for pp60^{c-SRC} (Schlessinger & Ullrich, 1992). These amino acid residues are presumably important for binding to intracellular signaling protein (Kavanaugh et al., 1995). The nucleotide sequence analysis of human genomic DNA fragments of the HEP gene revealed the presence of two introns between subdomains I and IX. The 3' intron (0.7 kb) was located in the deleted 14-amino acid stretch. However, no corresponding amino acid sequence was found in the intron, using any of the three possible reading frames. Consequently, this deletion was not caused by a error on splicing.

### Histological distribution of the HEP receptor

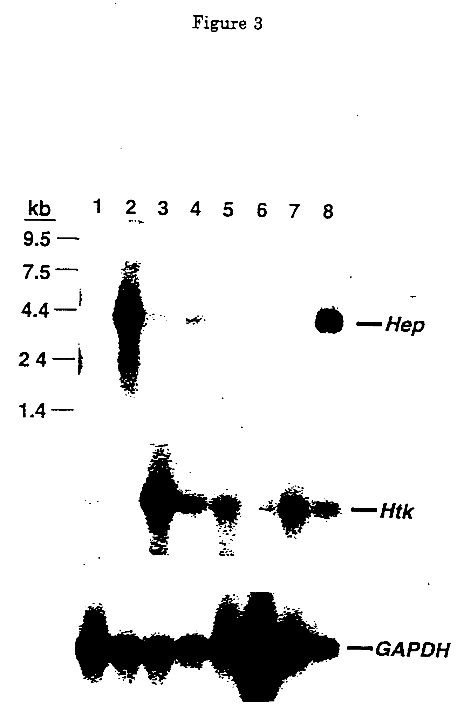

It was found out that the expression of the HEP receptor was very strong in the brain and pancreas, and it was weak in other tissues such as heart, placenta, lung, liver, skeletal muscle and kidney by an RNA blot analysis for a histological distribution of the HEP receptor (Figure 3). The HEP receptor protein was detected in the normal human brain, normal human peripheral blood mononuclear cells, human leukemic cells (HPB-MLT cells) by an immunoprecipitation method using an antibody against the HEP receptor protein and an immunoblot method.

It was found out from these results that the HEP receptor is expressed widely in normal tissues. It is remarkable that the HEP receptor is expressed widely in the normal tissues in consideration of the fact that the HEP receptor has an unique structure. This suggests that the HEP receptor takes part in some normal functions of cells.

It was conformed that the HEP receptor gene is located at a human chromosomal region 7q33→q35 by a human chromosomal mapping of the HEP receptor gene locus.

### Discussion on the HEP receptor's function

As described above, the deduced amino acid sequence of the HEP receptor gene has kinase subdomains and a conserved tyrosine residue which is a common structural feature among tyrosine-kinases. Additionally, the amino acid sequence shows very high homology to those of other Eph family receptor-type tyrosine-kinases. It is suggested from the deduced amino acid sequence that the HEP receptor is a membrane binding type receptor and, therefore, specifically controls a variety of cellular functions including cell proliferation and differentiation. Consequently, if a ligand of the HEP receptor was identified, it could be used for a screening of agonists or antagonists.

It is possible to give the following consideration about a function of the HEP receptor based upon its unique structure. Prudent nucleic acid sequencing on multiple independent HEP receptor cDNA clones form normal human brain, pancreas, and hemocytes exhibited that six of ten amino acids in kinase subdomains were substituted in the HEP receptor. These ten amino acids are conserved in all the other tyrosine-kinases and critical for the kinase activity. Especially, two sites, lysine at an ATP (adenosine triphosphate) binding site and aspartic acid at an transphosphorylation site, are substituted with glutamine and serine, respectively. It is therefore likely that the HEP receptor lacks kinase activity. Indeed, the following examples showed that the receptor lacks the kinase activity.

Several human diseases and mutant strains of mice have been shown to be due to various point mutations at the conserved amino acid residues of receptor-type tyrosine-kinases. For example, the point mutations at the conserved amino anid residues in kinase domain of the insulin receptor have been identified among some patients with diabetes mellitus (Moller & Flier, 1991). Invariant lysine in the ATP-binding fold, substituted with glutamine in the HEP receptor, has been shown to be critical for kinase activity (Hanks et al., 1988). Site-directed mutagenesis performed on this lysine in the ATP-binding fold of v-*Src*, v-*Mos*, v-*Fps*, EGF receptor, insulin receptor and PDGF β-receptor abolished their intrinsic tyrosine-kinase activities (Hanks et al., 1988 ; Escobedo et al., 1988). Similarly, aspartic acid in the phosphotransfer site of kinase subdomain VI, which is substituted with serine in HEP receptor, is invariant in all the known protein kinases (Hanks et al., 1988). Since a murine *W*^{*42*} mutation of the receptor type tyrosine-kinase c-kit receptor, in which only the equivalent aspartic acid in a phosphotransfer site was substituted with asparagine, is lethal in homozygous individuals and causes severe anemia in heterozygous individuals (Tan et al.,1990). This mutation abolished the ligand-dependent autophosphorylation of the *c-kit* receptor. Introduction of the "W⁴²"-type mutation at the identical aspartic acid in v-fps, c-fms, and murine flk2/flt3 abolished their kinase activities of receptor type tyrosine-kinases (Moran et al., 1988 ; Reith et al., 1993 ; Maroc et al., 1993). Recently, a member of EGF receptor family, erbB3, possessing three alterations in the invariant amino acid residues of the kinase domain has also been shown to lack an intrinsic kinase activity (Guy et al.,1994). These reports strongly support that the HEP receptor having the mutation described above lacks an kinase activity.

As shown in the following examples, an intrinsic tyrosine-kinase activity of the HEP receptor protein were examined. Since a specific ligand to the HEP receptor has not yet been identified, chimeric receptors possessing the ligand binding region of macrophage colony stimulating factor (C-FMS) receptor (FMS), that is, a chimeric receptor (FMS/HEP receptor) consisting of an extracellular region of FMS receptor and a cytoplasmic region of HEP receptor and a chimeric receptor (FMS/HTK receptor) consisting of an extracellular region of FMS receptor and a cytoplasmic region of HTK (Hepatoma transmembrane kinase) were prepared. In vitro kinase assay was performed by using these receptor proteins. An obvious phosphorylation of tyrosine residue were observed in FMS/HTK receptor and FMS receptor from the result. But a phosphorylation of tyrosine residue was not observed in FMS/HEP receptor . Conclusively, the HEP receptor is thought to lack its intrinsic tyrosine-kinase activity.

Most kinase-deficient forms of growth factor receptors have been described as a pathogenic receptor. For example, a kinase activity-deficient insulin receptor causes diabetes mellitus, and a kinase activity-deficient c-kit receptor causes human or W mice piebaldism (Moller et al., 1991; Giebel et al., 1991; Tan et al., 1990). Artificially constructed mutants of FGF receptor or Tek receptor resulted in defective gastrulation or vasculogenesis, respectively (Amaya et al., 1991; Dumont et al., 1994). Although erbB3 lacking an intrinsic tyrosine-kinase activity was reported to be expressed in normal human epithelial tissues and brain, it is rather overexpressed in several human malignancies (Kraus et al., 1989; Altiok et al., 1995; Moscoso et al., 1995). And, it is thought to play a key role in tumorigenesis (Kraus et al., 1993; Alimandi et al., 1995). Very recent, two Flt/Flk-like receptor genes, Klg (kinase-like gene) and Cck-4 (colon carcinoma kinase-4) also have been found to lack a kinase activity due to substitution in the conserved amino acid sequence of a kinase region (Chou & Hayman, 1991 ; Mossie et al.,1995). These genes are also reported to be preferentially expressed in transformed or tumor cells, but little in normal human tissues. The HEP receptor is abundantly expressed in normal human tissues including the brain where many other Eph-family tyrosine-kinase receptors are simultaneously expressed. Thus, it is very important to elucidate the physiological function of the HEP receptor.

Kinase activity-deficient mutant type receptors may show a dominant-negative effect (superiority of mutant type) by heterodimerization with a wild-type receptor. For example, several phenotypes of W mice which show similar symptoms to human congenital piebaldness characterized by leucoderma and canities were reported to be caused by a dominant-negative effect of mutant type c-Kit receptor in which an invariant amino acid residue in all tyrosine kinase receptors has been substituted (Tan et al., 1990). In c-fms-, PDGF β-, insulin- and Tek-mutant receptors, the replacements of the lysine in the ATP-binding site and the aspartic acid in the phosphotransfer site observed in HEP receptor are enough to make them dominant-negative type receptors (Ray et al., 1991 ; Reith et al., 1993 ; Ueno et al., 1993 ; Grako et al., 1994 ; Dumont et al., 1994). These results of prior observation suggest that the HEP receptor having a similar structural mutation may take part in signal transmission of Eph family as a dominant-negative type receptor.

Another HEP receptor function can be thought from a recent report on erbB3 of EGF receptor family (Carraway & Cantley, 1994). The erbB3 could be transphosphorylated by heterodimerization with other members of the EGF receptor family, providing docking sites for SH2 proteins not recruited by the autophosphorylated kinase-active receptors. Therefore, erbB3 is thought to be an amplifier or modulator for Eph family receptors (Alimandi et al., 1995). Three amino acid residues of invariant ones are substituted in kinase domains of erbB3, which lacks an intrinsic tyrosine-kinase activity. Therefore, there is a possibility that the HEP receptor is also an amplifier or modulator for other Eph family receptors.

Very recently, most Eph-family ligands and their receptors were shown to be divided into two major subclasses from the viewpoint of their ligand-binding specificity (Gale et al., 1996). Each of ligands to the Eph family can bind to multiple receptor isoforms belonging to an identical subclass (Brambilla et al., 1995). Therefore, the ligands of the HEP receptor may bind to other Eph family receptors such as Elk, Erk/Cek5, and Cek9 because the amino acid sequence of the HEP receptor in the extracellular region was well conserved among these Eph family receptors.

### Preparation of Eph family receptor type tyrosine kinase like protein lacking kinase activity

If a DNA molecule encoding the HEP receptor described above was obtained, the HEP receptor could be expressed by using usual methods in the field of genetic engineering. For example, the obtained DNA molecule is inserted into an expression vector, host cells are transformed by this vector according to usual methods, and the obtained transformants are cultured under an appropriate condition. Examples of a host cell are not only animal and insect cells, but also Escherichia coli and yeast. CHO-K1 cell etc. are given as an example of an animal cell.

The other proteins of the present invention are able to be produced by mutating the amino acid sequence (substitution, deletion and addition) of HEP receptor. As methods of the mutation, a site-specific mutagenesis inducing method by PCR method using a synthesized oligonucleotide and other methods well known by skilled persons in the art are exemplified.

### Example

### Example 1

### Cloning of DNA molecule encoding HEP receptor

cDNA libraries were constructed from human brain and hematopoietic cells with λ gt 10 phage as described in Ito et al., 1993, and 1 x 10⁶ independent clones were isolated. The obtained clones were transferred to a nitrocellulose filter and DNA was denatured by alkali. After that, the screening was performed by using a ³²P-labeled cDNA probe corresponding to the kinase domain (nucleotides 1803-2907) (Bennett et al., 1994) of human Htk (Hepatoma transmembrane kinase) (Bennet et al., 1994) and 27 λ phage clones were isolated. Hybridizations were performed in a buffer containing 35 % W/V formamide, 5 x SSC (1 x SSC is 0.15 M NaCl - 0.015 M sodium citrate, pH 7.0), 1 x Denhardt's solution, 5 mM NaH₂PO₄, 0.1 % SDS and 100 µg/ml salmon sperm DNA at 42 °C for 18 h (Matsui et al., 1989). cDNA size inserted in all 27 λ phage clones was analyzed by electrophoresis, and nucleotide sequences of 5 clones among them having obviously different size were determined from both strands by using Sequenase Version2.0 (United States Biochemical, Cleveland, OH). The sequences were analyzed then using BLAST program (the Institute for Chemical Research, Kyoto University). The result is shown in Figure 1. Nucleotide sequence analyses revealed that the φ 49 clone contained an open reading frame of 3,018 nucleotides encoding 1,006 amino acids, flanked by a 5'-untranslated region of 798 nucleotides and a 3'-untranslated region of 191 nucleotides. The other four clones had restriction enzyme sites and nucleotide sequences that were identical to those of the φ 49 clone in the overlapping region (Figure 1).

### Example 2

### Isolation of HEP receptor gene and pancreas origin cDNA

PCR was performed on human genomic DNA using the following primers flanking the juxtamembrane domain and kinase subdomain X of the φ 49 clone:
T CAG GCC ATC CGA GAA CTT GCC CG (sense) [SEQ ID NO. 3];
CAT GTC CCA GTA AGG CCG TTC TCC A (antisense) [SEQ ID NO. 4]

The PCR was performed by using Taq DNA polymerase (Promega) and repeating a cycle of 95 °C; 5 min, 94 °C; 30 sec, 60 °C; 30 sec, and 72 °C; 2 min 95 times. Reverse transcriptase-polymerase chain reaction(RT-PCR) was performed on human pancreas RNA using the same primers. RT-PCR was performed under the same condition as above except for using reverse transcriptase produced by GIBCO-BRL. Then, human genomic DNA and cDNA fragments produced from pancreas RNA by using reverse transcriptase were sequenced by the method described above. These sequences are identical with that of the cDNA obtained in Example 1.

### Example 3

### Histological distribution of HEP receptor

Two µ g polyadenylized RNA obtained from the eight tissues, heart, brain, placenta, lung, liver, skeletal muscle, kidney and pancreas, were blotted for a human Multiple Tissue Northern (MTN) blot (Clontech Laboratories, Palo Alto, Ca). The blots were hybridized with the ³²P-labeled HEP receptor cDNA probes according to the manufacturer's protocol. As a control, Htk cDNA probe and human glyceraldehyde-3-phosphate dehydrogenase probe were also hybridized.

The obtained blots were washed twice in 2 x SSC/0.05 % SDS at room temperature for 20 min, then washed twice in 0.1 x SSC/0.1 % SDS at 50 °C for 20 min (Matusi et al., 1989), and subjected to autoradiography. The result is shown in Figure 3.

From the RNA blot analysis of polyadenylized RNA obtained from the eight human adult normal tissues, 4.0 kb size single bands corresponding to the φ 49 cDNA clone were found out in all the tissues. Expression was strong in the brain and pancreas (Figure 3, lanes 2 and 8), but weak in other tissues such as the heart, placenta, lung, liver, skeletal muscle and kidney (Figure 3, lanes 3, 4, 5, 6 and 7). This broad expression of the present invention's gene in human normal tissues suggests that a product of the gene functions in normal tissues. The Htk gene used for the HEP receptor cloning showed a different expression pattern from the HEP gene.

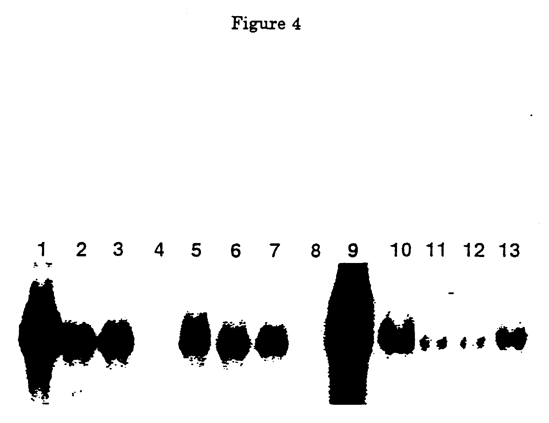

In the same manner, Northern blotting was performed on ① cerebrum of embryo, ② retina, ③ thyroid gland, ④ adrenal gland, ⑤ testes, ⑥ bronchus, ⑦ salivary glands, ⑧ small intestine, ⑨ thymus gland, 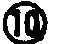 human peripheral blood leukocytes, 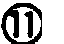 bone marrow cell, 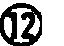 CD34 negative hematopoietic stem cell, and 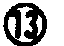 CD34 positive hematopoietic stem cell. The result is shown in Figure 4 (the above number of each tissue coincides with the lane number in Figure 4).

Figure 4 shows that HEP receptor is strongly expressed in thymus gland, but slightly in small intestine. This result suggests that the HEP receptor plays an important role in not only the central nervous system but also the immune system, especially in the development and differentiation of T cells, and there is a possibility that it takes part in the nerve-immune network.

### Example 4

### Chromosomal hybridization

R-banded chromosomes were prepared by standard methods with some modification (Takai et al., 1994). Peripheral blood lymphocytes of a healthy male were stimulated by phytohemagglutinin (Welcome, HA15), and cultured in TC199 medium (GIBCO-BRL). After 48 h, thymidine (300 µ g/ml, Sigma) was added. After 15.5 h, the cells were washed in phosphate-buffered saline, and treated by bromodeoxyuridine (25 µ g/ml) for 6.5 h. Preparation of chromosomes were carried out according to the standard method (Takai, S et al., (1994) Hum. Genet., 93, 198-200). The slides were stained with Hoechst 33258 (1 µ g/ml), heated for 3 min, and exposed to 20 W black light (Toshiba, FL20SBLB, Japan) at 75 °C for 6 min. This exposed slides were washed with distilled water, and stored at -80°C until use.

As a probe for fluorescence in situ hybridization (FISH), 2.2 kb HEP receptor cDNA probe derived from the φ 8 clone was used. The probe was labeled by nick translation with biotin-16-dUTP (Boehringer Mannheim). Then, hybridization was performed according to the method described in Takai et al.,1994. Signal amplification was carried out with fluorescein avidin DCS (Cell Sorting grade, Vector Laboratory) and biotinylated anti-avidin goat antibody (Vector Laboratory). The obtained slides were stained with propidium iodide (0.5 µ g/ml). A Nikon OPTIPHOT-2-EFD2 microscope (B-2A filter) was used for observation. Kodak Ektachrome film (ASA 100) was used for photomicrography of chromosomes. Among fifty human (pro) metaphase cells hybridized with a 2.2kb HEP receptor cDNA probe described above, thirty-two cells revealed symmetrical double spots on at least one copy of chromosome 7 at band q33→q35. Three cells showed symmetrical double spots on both chromosomes 7 in each cell at the same region. No symmetrical double spots were detected in other regions with any coincidence. Thus, it is concluded that the HEP gene is located at human chromosomal region 7q33→q35.

### Example 5

### Expression of the HEP receptor protein in CHO-K1 cells

A 3.3-kilobase fragment containing the entire coding region of HEP receptor cDNA (nucleotides 658 - 4022) was cloned into the pH β APrlneo expression vector (Gunning et al., 1987). Approximately 30,000 CHO-K1 cells in 6-cm dishes were transfected with 10 µ g of plasmid DNAs by the Chen-Okayama method (Mol. Cell. Biol. 7, 2745 - 2752 (1987)). Then, G418-resistant cells were selected in the presence of 700 µ g/ml Geneticin (GIBCO) (Ito et al., 1993).

### Example 6

### Confirmation of the HEP receptor protein expression in human normal tissues by immunoprecipitation and immunoblot analyses

A rabbit antiserum was obtained by immunizing rabbits with the peptide (LHHIQLLQQHLRQQGSVEV) based the C-terminal amino acid sequence of HEP receptor deduced from the cDNA coupled to keyhole limpet hemocyanin by using glutaraldehyde or with the peptide expressed in *E. coli* using cDNA of the HEP receptor intracellular region (from 577th arginine to 1006th valine in SEQ ID NO.1).

Normal human brain tissues, normal human peripheral blood mononuclear cells, and human T cell leukemic cells (HPB-MLT cell) each was washed twice with PBS (phosphate-buffered saline) and scraped in lysis buffer containing 50 mM HEPES (pH 7.4), 1 % Triton X-100, 20 mM NaCl, 5 mM EDTA, 2 mM sodium vanadate, 10 mM sodium pyrophosphate, 25 mM sodium fluoride, 0.02 % sodium azide, 1 mM phenylmethylsulfonyl fluoride, 16.5 µ g/ml aprotinin, 1.0 µ g/ml leupeptin, and 1.25 µ g/ml pepstatin. Cell lysates were clarified by centrifugation at 14,000 x g for 30 min, and pre-cleared with preimmune serum and protein-G-Sepharose (Pharmacia) before immunoprecipitation with anti-HEP serum.

Immunoprecipitates brought down with protein-G-Sepharose or total cell lysates were resolved by SDS-polyacrylamide gel electrophoresis, transferred onto Immobilon (Millipore) filters, and probed by immunoblot analysis using ¹²⁵I-protein A.

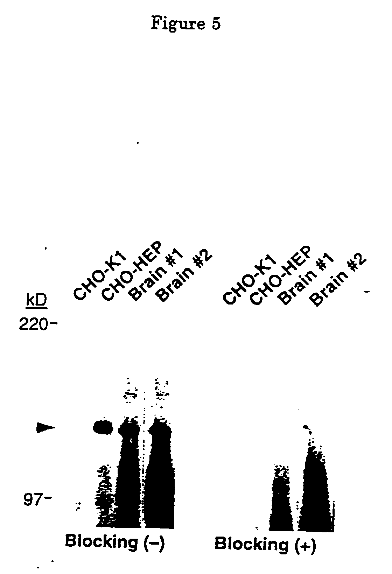

Cell lysates of a wild type CHO-K1 cell (Figure 5, CHO-K1), a transfectant of CHO-K1 cell having a full length HEP cDNA expression vector (Figure 5, CHO-HEP), and normal human brain (Figure 5, Brain #1 and Brain #2) were first immunoprecipitated by a rabbit anti-peptide serum against the HEP specific peptide (C-terminus). Immunoblotting with the serum was performed in the presence (Figure 5, Blocking (+)) or absence (Figure 5, Blocking (-)) of the peptide used as immunogen.

From the result shown in Figure 5, the HEP receptor protein was detected by the anti-serum at 135 KD position (Figure 5, the band shown by 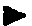 ) in the CHO-K1 cell transfectant, but not in the wild type CHO-K1 cell. The 135 KD band specifically disappeared in the presence of the peptide. Additionally, the HEP receptor protein was immunoprecipitated from the human brain by the anti-serum. These results strongly support that products of the HEP receptor gene are expressed in human normal tissues.

A HEP receptor protein having the same molecular weight 135 KD and expressed in mouse brain tissue, human peripheral blood mononuclear cells, and human leukemic cells was detected by immunoblotting with a rabbit anti-serum against the antigen, the intracellular region protein.

A mouse monoclonal antibody against the HEP receptor extracellular region (from 23rd aspartic acid to 576th glutamic acid in SEQ ID No.1) was prepared and the HEP receptor proteins in normal mouse brain, normal human peripheral blood mononuclear cells, and HPB-MLT cells were detected by immunoblotting or flow cytometory using the antibody.

### Example 7

### Investigation of HEP receptor's intrinsic tyrosine kinase activity

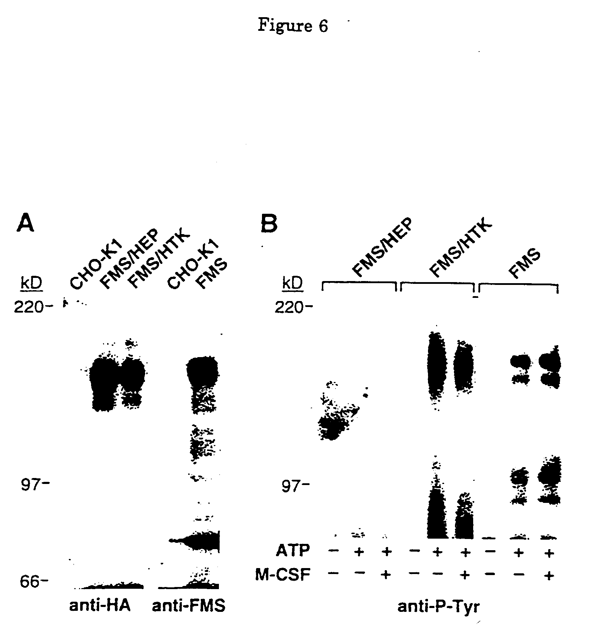

(1) Chimeric receptors, FMS/HEP and FMS/HTK, in which the extracellular regions of HEP receptor and Htk receptor were replaced with that of macrophage colony-stimulating factor (M-CSF) receptor (FMS) were prepared.
   To create a FMS/HEP chimera receptor cDNA, a EspEI site was created at nucleotide 1842 of FMS receptor cDNA (Genbank accession No. X03663) by using PCR methods. Then, the fragement encoding the extracellular region of HEP receptor was replaced with that of FMS at the BspEI site (nucleotide 2520 of HEP receptor cDNA). Similarly, EcoRI sites were introduced at nucleotide 1834 of FMS receptor cDNA and at nucleotide 1690 of Htk receptor cDNA (Genbank accession No. U07695). Then, the fragment coding the extracellular region of Htk receptor was replaced with that of FMS receptor at the EcoRI site. Furthermore, the influenza virus hemaglutinin (HA) epitope sequences were added at the end of each coding sequence. Finally, nucleotide sequences of all PCR products were confirmed. The nucleotide sequences of the chimeric receptors were designed to code for amino acid sequences that conserved those of native receptors in each corresponding region. The chimeric receptors were equally expressed in CHO-K1 cells.
(2)In Vitro Kinase Assays
   These receptor proteins were prepared from CHO cells in which expression vectors of chimeric receptor cDNAs described in above (1) and FMS receptor cDNA of full length were introduced and expressed, respectively. Wild type CHO-K1 cells (CHO-K1 in Figure 6) and two transfectants with the chimeric receptor cDNA expression vectors having an HA amino acid sequence at the C-terminus (FMS/HEP and FMS/HTK in Figure 6) were first subjected to immunoprecipitation with an anti-HA monoclonal antibody (12CA5; Boehringer Mannheim) to confirm the expression. After electrophoresis, the immunoprecipitates were subjected to an immunoblot analysis by using the anti-HA monoclonal antibody (Figure 6, left panel).

Wild type CHO-K1 cells and FMS receptor cDNA transfectant (Figure 6, FMS) were immunoblotted with an anti-FMS antiserum (Figure 6, right panel). An anti-FMS receptor antiserum was obtained after immunization of rabbits with FMS receptor protein (VAKIGDFGLARDIM).

From the result shown in Figure 6A, each of the chimeric receptors and full length FMS receptor was well expressed.

Then, the immunoprecipitates (FMS/HEP chimeric receptor protein and FMS/HTK chimeric receptor protein) by the anti-HA monoclonal antibody and the immunoprecititates (FMS receptor protein) by an anti-FMS monoclonal antibody (3-4A4; Santa Cruz Biotechnology) were washed twice with a kinase buffer containing 25 mM HEPES (pH 7.4), l mM dithiothreitol, 10 mM MgCl₂, and 10 mM MnCl₂. Pellets were incubated in 40 µ l of the kinase buffer supplemented with or without 20 µ M ATP. After a 10 min incubation at 30 °C in the presence or absence of human recombinant macrophage colony stimulating factor (M-CSF) (100 ng/ml), reactions were stopped by boiling for 3 min in 40 µ l of 2 x Laemmli buffer, and subjected to immunoblot analysis. Tyrosine-phosphorylated receptors were probed with anti-phosphotyrosine monoclonal antibody, PY-20 (ICN Biomedicals,Costa Mesa,CA) as described in Tsukamoto, T. et al., J. Biol. Chem., 266: 10143-10147 (1991).

From the result shown in Figure 6B, although definite tyrosine-phosphorylation of FMS/HTK and FMS receptors was observed in the presence of ATP, no tyrosine-phosphorylation was detectable in the FMS/HEP fusion protein. A ligand-dependent increase in tyrosine-phosphorylation was induced in FMS but not in FMS/HEP or FMS/HTK receptor. These results are consistent with previous reports that soluble ligand(s) for HTK receptor including Eph-family receptors could not sufficiently induce the receptor auto-phosphorylation as did their native membrane-bound ligand(s) (Bennett et al., 1995; Brambilla et al., 1995). Conclusively the HEP receptor is thought to lack its intrinsic tyrosine kinase activity.

### Effect of the Invention

The kinase-defective receptor protein of this invention, which is well expressed in human normal tissues, contributes to the elucidation of the mechanism of normal cell function control by Eph family receptor type tyrosine kinase.

As described above, HEP receptor is a membrane-binding receptor and is thought to specifically control cell functions. Therefore, if HEP receptor ligand(s) were determined, it could be used for the screening for an agonist or antagonist.

Furthermore, a DNA molecule encoding HEP receptor can be used as a probe. Receptors having unique structure like HEP receptor can be found out by PCR methods using the DNA molecule of the present invention.

### References

- Aaronson SA. (1991). Science, 254, 1146-1153.
- Alimandi M, Romano A, Curia MC, Muraro R, Fedi P, Aaronson SA, Di Fiore PP and Kraus MH. (1995). Oncogene, 1 0, 1813-1821.
- Altiok N, Bessereau JL and Changeux JP. (1995). EMBO J., 14, 4258-4266
- Amaya E, Musci TJ and Kirschner MW. (1991). Cell, 66, 257 -270
- Bennett BD, Wang Z, Kuang WJ, Wang A, Groopman JE, GoeddelDV and Scadden DT. (1994). J. Biol. Chem., 269, 14 211-14218.
- Brambilla R, Schnapp A, Casagranda F, Labrador JP, BergemannAD, Flanagan JG, Pasquale EB and Klein R. (1995). E MBO J.,14, 3116-3126.
- Carraway III KL and Cantley LC. (1994). Cell, 78, 5-8.
- Chou YH and Hayman MJ. (1991). Proc. Natl. Acad. Sci. USA,88, 4897-4901.
- Davis S, Gale NW, Aldrich TH, Maisonpierre PC, Lhotak V, Pawson T, Goldfarb M and Yancopoulos GD. (1994). Science,266, 816-819.
- Dumont DJ, Gradwohl G, Fong GH, Furi MC, Gertsenstein M,Auerbach A and Breitman ML. (1994). Genes Dev., 8, 189 7-1909.
- Escobedo JA, Barr PJ and Williams LT. (1988). Mol. Cell.Biol., 8, 5126-5131.
- Gale NW, Holland SJ, Valenzuela DM, Flenniken A, Pan L, Ryan TE, Henkemeyer M, Strebhardt K, Hirai H, Wilkinson DG,Pawson T, Davis S and Yancopoulos GD. (1996), Neuron , 17, 9-19.
- Giebel LB and Spritz RA. (1991), Proc. Natl. Acad. Sci. USA,88, 8696-8699.
- Grako KA, McClain DA and Olefsky JM. (1994). Mol.Endocrinol.,8, 682-692.
- Gunning P, Leavitt J, Muscat G, Ng SY and Kedes L. (198 7). Proc. Natl. Acad. Sci. USA, 84, 4831-4835.
- Guy PM, Platko JV, Cantley LC, Cerione RA and Carraway III
- KL. (1994). Proc. Natl. Acad. Sci. USA, 91, 8132-8136.
- Hanks SK, Quinn AM and Hunter T. (1988). Science, 241,42 -52.
- Hunter T. (1991). Cell, 64, 249-270.
- Hirai H, Maru Y, Hagiwara K, Nishida J and Takaku F.(1 987).Science, 238, 1717-1720.
- Ito M, Matsui T, Taniguchi T, Tsukamoto T, Murayama T, ArimaN, Nakata H, Chiba T and Chihara K. (1993). J. Biol. Chem.,268, 18300-18305.
- Kavanaugh WM, Turck CW and Williams LT. (1995). Science, 268,1177-1179.
- King CR, Kraus MH and Aaronson SA. (1985). Science, 229, 974-976.
- Kozak M. (1984). Nucleic Acids Res., 12, 857-872.
- Krauz MH, Fedi P, Starks V, Muraro R and Aaronson SA. ( 1993). Proc. Natl. Acad. Sci. USA, 90, 2900-2904.
- Maroc N, Rottapel R, Rosnet O, Marchetto S, Lavezzi C, M annoni P, Birnbaum D and Dubreuil P. (1993). Oncogene, 8, 909-918.
- Matsui T, Heidaran M, Miki T, Popescu N, La Rochelle W, KrausM, Pierce J and Aaronson SA. (1989). Science, 243, 800-804.
- Moller DE and Flier JS. (1991). N. Engl. J. Med., 325,938-948.
- Moran MF, Koch CA, Sadowski I and Pawson T. (1988). Oncogene, 3, 665-672.
- Moscoso LM, Chu GC, Gautam M, Noakes PG, Merlie JP and Sanes JR. (1995). Dev. Biol., 172, 158-169.
- Mossie K, Jallal B, Alves F, Sures I, Plowman GD and Ullrich A. (1995). Oncogene, 11, 2179-2184.
- Ray P, Higgins KM, Tan JC, Chu TY, Yee NS, Nguyen H, Lacy E and Besmer P. (1991). Genes Dev., 5, 2265-2273.
- Reith AD, Ellis C, Maroc N, Pawson T, Bernstein A and Dubreuil P. (1993). Oncogene, 8, 45-53.
- Schlessinger J and Ullrich A. (1992). Neuron, 9, 383-391.
- Shibuya M, Yamaguchi S, Yamane A, Ikeda T, Tojo A, Matsushime and Sato M. (1990). Oncogene, 5, 519-524.
- Takai S, Yamada K, Hirayama N, Miyajima A and Taniyama T.(1994). Hum. Genet., 93, 198-200.
- Tan JC, Nocka K, Ray P, Traktman P and Besmer P. (19 90).Science, 247, 209-212.
- Tuzi NL and Gullick WJ. (1994). Br. J. Cancer, 69, 417-421.
- Ueno H, Escobedo JA and Williams LT. (1993). J. Biol. Chem.,268, 22814-22819
- Ullrich A and Schlessinger J. (1990). Cell, 61, 203-212.
- Von Heijne G. (1986). Nucleic Acids Res., 14, 4683-4690.
- Wilks AF. (1989). Proc. Natl. Acad. Sci. USA, 86, 1603-1607
- Yamamoto T, Ikawa S, Akiyama T, Semba K, Nomura N, Miyajima N, Saito T and Toyoshima K. (1986). Nature, 319, 230-234.

SEQUENCE LISTING

SEQ ID NO : 1
Length : 1,006 amino acids
Type : amino acid
Topology : linear
Kind : peptide
Sequence :
Met Val Cys Ser Leu Trp Val Leu Leu Leu Val Ser Ser Val Leu Ala
1               5                   10                  15
Leu Glu Glu Val Leu Leu Asp Thr Thr Gly Glu Thr Ser Glu Ile Gly
            20                  25                  30
Trp Leu Thr Tyr Pro Pro Gly Gly Trp Asp Glu Val Ser Val Leu Asp
        35                  40                  45
Asp Gln Arg Arg Leu Thr Arg Thr Phe Glu Ala Cys His Val Ala Gly
    50                  55                  60
Ala Pro Pro Gly Thr Gly Gln Asp Asn Trp Leu Gln Thr His Phe Val
65 ·70 75 80
Glu Arg Arg Gly Ala Gln Arg Ala His Ile Arg Leu His Phe Ser Val
85 90 95
Arg Ala Cys Ser Ser Leu Gly Val Ser Gly Gly Thr Cys Arg Glu Thr
100 105 110
Phe Thr Leu Tyr Tyr Arg Gln Ala Glu Glu Pro Asp Ser Pro Asp Ser
115 120 125
Val Ser Ser Trp His Leu Lys Arg Trp Thr Lys Val Asp Thr Ile Ala
130 135 140
Ala Asp Glu Ser Phe Pro Ser Ser Ser Ser Ser Ser Ser Ser Ser Ser
145 150 155 160
Ser Ala Ala Trp Ala Val Gly Pro His Gly Ala Gly Gln Arg Ala Gly

165 170 175
Leu Gln Leu Asn Val Lys Glu Arg Ser Phe Gly Pro Leu Thr Gln Arg
180 185 190
Gly Phe Tyr Val ala Phe Gln Asp Thr Gly Ala Cys Leu Ala Leu Val
195 200 205
Ala Val Arg Leu Phe Ser Tyr Thr Cys Pro Ala Val Leu Arg Ser Phe
210 215 220
Ala Ser Phe Pro Glu Thr Gln Ala Ser Gly Ala Gly Gly Ala Ser Leu
225 230 235 240
Val Ala Ala Val Gly Thr Cys Val Ala His Ala Glu Pro Glu Glu Asp
245 250 255
Gly Val Gly Gly Gln Ala Gly Gly Ser Pro Pro Arg Leu His Cys Asn
260 265 270
Gly Glu Gly Lys Trp Met Val Ala Val Gly Gly Cys Arg Cys Gln Pro
275 280 285
Gly Tyr Gln Pro Ala Arg Gly Asp Lys Ala Cys Gln Ala Cys Pro Arg
290 295 300
Gly Leu Tyr Lys Ser Ser Ala Gly Asn Ala Pro Cys Ser Pro Cys Pro
305 310 315 320
Ala Arg Ser His Ala Pro Asn Pro Ala Ala Pro Val Cys Pro Cys Leu
325 330 335
Glu Gly Phe Tyr Arg Ala Ser Ser Asp Pro Pro Glu Ala Pro Cys Thr
340 345 350
Gly Pro Pro Ser Ala Pro Gln Glu Leu Trp Phe Glu Val Gln Gly Ser
355 360 365
Ala Leu Met Leu His Trp Arg Leu Pro Arg Glu Leu Gly Gly Arg Gly
370 375 380
Asp Leu Leu Phe Asn Val Val Cys Lys Glu Cys Glu Gly Arg Gln Glu
385 390 395 400

Pro Ala Ser Gly Gly Gly Gly Thr Cys His Arg Cys Arg Asp Glu Val
405 410 415
His Phe Asp Pro Arg Gln Arg Gly Leu Thr Glu Ser Arg Val Leu Val
420 425 430
Gly Gly Leu Arg Ala His Val Pro Tyr Ile Leu Glu Val Gln Ala Val
435 440 445
Asn Gly Val Ser Glu Leu Ser Pro Asp Pro Pro Gln Ala Ala Ala Ile
450 455 460
Asn Val Ser Thr Ser His Glu Val Pro Ser Ala Val Pro Val Val His
465 470 475 480
Gln Val Ser Arg Ala Ser Asn Ser Ile Thr Val Ser Trp Pro Gln Pro
485 490 495
Asp Gln Thr Asn Gly Asn Ile Leu Asp Tyr Gln Leu Arg Tyr Tyr Asp
500 505 510
Gln Ala Glu Asp Glu Ser His Ser Phe Thr Leu Thr Ser Glu Thr Asn
515 520 525
Thr Ala Thr Val Thr Gln Leu Ser Pro Gly His Ile Tyr Gly Phe Gln
530 535 540
Val Arg Ala Arg Thr Ala Ala Gly His Gly Pro Tyr Gly Gly Lys Val
545 550 555 560
Tyr Phe Gln Thr Leu Pro Gln Gly Glu Leu Ser Ser Gln Leu Pro Glu
565 570 575
Arg Leu Ser Leu Val Ile Gly Ser Ile Leu Gly Ala Leu Ala Phe Leu
580 585 590
Leu Leu Ala Ala Ile Thr Val Leu Ala Val Val Phe Gln Arg Lys Arg
595 600 605
Arg Gly Thr Gly Tyr Thr Glu Gln Leu Gln Gln Tyr Ser Ser Pro Gly
610 615 620
Leu Gly Val Lys Tyr Tyr Ile Asp Pro Ser Thr Tyr Glu Asp Pro Cys

625 630 635 640
Gln Ala Ile Arg Glu Leu Ala Arg Glu Val Asp Pro Ala Tyr Ile Lys
645 650 655
Ile Glu Glu Val Ile Gly Thr Gly Ser Phe Gly Glu Val Arg Gln Gly
660 665 670
Arg Leu Gln Pro Arg Gly Arg Arg Glu Gln Thr Val Ala Ile Gln Ala
675 680 685
Leu Trp Ala Gly Gly Ala Glu Ser Leu Gln Met Thr Phe Leu Gly Arg
690 695 700
Ala Ala Val Leu Gly Gln Phe Gln His Pro Asn Ile Leu Arg Leu Glu
705 710 715 720
Gly Val Val Thr Lys Ser Arg Pro Leu Met Val Leu Thr Glu Phe Met
725 730 735
Glu Leu Gly Pro Leu Asp Ser Phe Leu Arg Gln Arg Glu Gly Gln Phe
740 745 750
Ser Ser Leu Gln Leu Val Ala Met Gln Arg Gly Val Ala Ala Ala Met
755 760 765
Gln Tyr Leu Ser Ser Phe Ala Phe Val His Arg Ser Leu Ser Ala His
770 775 780
Ser Val Leu Val Asn Ser His Leu Val Cys Lys Val Ala Arg Leu Gly
785 790 795 800
His Ser Pro Gln Gly Pro Ser Cys Leu Leu Arg Trp Ala Ala Pro Glu
805 810 815
Val Ile Ala His Gly Lys His Thr Thr Ser Ser Asp Val Trp Ser Phe
820 825 830
Gly Ile Leu Met Trp Glu Val Met Ser Tyr Gly Glu Arg Pro Tyr Trp
835 840 845
Asp Met Ser Glu Gln Glu Val Leu Asn Ala Ile Glu Gln Glu Phe Arg
850 855 860

Leu Pro Pro Pro Pro Gly Cys Pro Pro Gly Leu His Leu Leu Met Leu
865                 870                 875                 880
Asp Thr Trp Gln Lys Asp Arg Ala Arg Arg Pro His Phe Asp Gln Leu
                885                 890                 895
Val Ala Ala Phe Asp Lys Met Ile Arg Lys Pro Asp Thr Leu Gln Ala
            900                 905                 910
Gly Gly Asp Pro Gly Glu Arg Pro Ser Gln Ala Leu Leu Thr Pro Val
        915                 920                 925
Ala Leu Asp Phe Pro Cys Leu Asp Ser Pro Gln Ala Trp Leu Ser Ala
    930                 935                 940
Ile Gly Leu Glu Cys Tyr Gln Asp Asn Phe Ser Lys Phe Gly Leu Cys
945                 950                 955                 960
Thr Phe Ser Asp Val Ala Gln Leu Ser Leu Glu Asp Leu Pro Ala Leu
                965                 970                 975
Gly Ile Thr Leu Ala Gly His Gln Lys Lys Leu Leu His His Ile Gln
            980                 985                 990
Leu Leu Gln Gln His Leu Arg Gln Gln Gly Ser Val Glu Val
        995                 1000                1005

SEQ ID NO : 2
Length : 4,022 nucleic acids
Type : nucleic acid
Strandedness : double
Topology : linear
Kind : cDNA
Sequence
CGGAGGGGGC GGGCCGGGCT GCGTTCGCTC CAGCCGCGGC TCTACAGCAG CGGGCGGCGG   60
GACCCGGGAC CCAGCTTGGC GACGGCGATT CTCGACGCGG GCCCCCAGGA TTCTCCCGGC  120
GCCCCACCTC TGGAGCAGCC CCTGCCGCCA GCGTCAGGTC CACCCCGGAA TCCCAGGGAC  180

TCTCGGCGCC GAACGGACCC GGGCCGGTGC AACGGGGTCC CCGGACTGGA GAAGACGCGG 240
GTGGCACCGT GCGAGCTCCA GGAGCCCCGG GTCCACTGCG AGGCCTCGGG GGGCGCAGAC 300
CTGCAGAGAC TGCGGCCAAC GGGAAGAAAT AAAGGGATTA TAGTCCACCC AATTCACAGA 360
CTTCTGAGAC TCAGACACGA GGAGAGATAG AGAACCGCCA ATCTCTAGAT CAACAAGCAA 420
AGGAGGTGCC AAGCCTGTTT GTCTTCATTG TGACACTGGA GTCTAGATGC TGGGAAGTCC 480
AAGATCAGGG TGCCGGCATG GTCAGTTCCT GGCGAAGCCT CTCTTCTAGG TTTCAGACTG 540
CCCTCTTCTT TGTTGTGTCC TCGAATGGCA GAAAAAGGGG TGGCTGTTGG AGGAAGGGAG 600
GAGAGTAAAT GAAGAGAAAG AACTGGAATA ACCCCTTGCA GAAAAAAAAA AAAAGGGAAG 660
CTTAGCTGTA CACCCTGAGT CTTGCAAAAG CTGCAGCCCC ACCCAGGAGC AGGGTGGTGG 720
CTGGGGCGAT GGTGGACGCC CTGAAGATGT CCCATGGCTA CTGAAGGGGC TGCCCAGTTA 780
GGGAACAGAG TGGCGGGC 798
ATG GTG TGT AGC CTA TGG GTG CTG CTC CTG GTG TCT TCA GTT CTG GCT 846
CTG GAA GAG GTA TTG CTG GAC ACC ACC GGA GAG ACA TCT GAG ATT GGC 894
TGG CTC ACC TAC CCA CCA GGG GGG TGG GAC GAG GTG AGT GTT CTG GAC 942
GAC CAG CGA CGC CTG ACT CGG ACC TTT GAG GCA TGT CAT GTG GCA GGG 990
GCC CCT CCA GGC ACC GGG CAG GAC AAT TGG TTG CAG ACA CAC TTT GTG 1038
GAG CGG CGC GGG GCC CAG AGG GCG CAC ATT CGA CTC CAC TTC TCT GTG 1086
CGG GCA TGC TCC AGC CTG GGT GTG AGC GGC GGC ACC TGC CGG GAG ACC 1134
TTC ACC CTT TAC TAC CGT CAG GCT GAG GAG CCC GAC AGC CCT GAC AGC 1182
GTT TCC TCC TGG CAC CTC AAA CGC TGG ACC AAG GTG GAC ACA ATT GCA 1230
GCA GAC GAG AGC TTT CCC TCC TCC TCC TCC TCC TCC TCC TCT TCT TCC 1278
TCT GCA GCG TGG GCT GTG GGA CCC CAC GGG GCT GGG CAG CGG GCT GGA 1326
CTG CAA CTG AAC GTC AAA GAG CGG AGC TTT GGG CCT CTC ACC CAA CGC 1374
GGC TTC TAC GTG GCC TTC CAG GAC ACG GGG GCC TGC CTG GCC CTG GTC 1422
GCT GTC AGG CTC TTC TCC TAC ACC TGC CCT GCC GTG CTC CGA TCC TTT 1470
GCT TCC TTT CCA GAG ACG CAG GCC AGT GGG GCT GGG GGG GCC TCC CTG 1518
GTG GCA GCT GTG GGC ACC TGT GTG GCT CAT GCA GAG CCA GAG GAG GAT 1566
GGA GTA GGG GGC CAG GCA GGA GGC AGC CCC CCC AGG CTG CAC TGC AAC 1614
GGG GAG GGC AAG TGG ATG GTA GCT GTC GGG GGC TGC CGC TGC CAG CCT 1662

GGA TAC CAA CCA GCA CGA GGA GAC AAG GCC TGC CAA GCC TGC CCA CGG 1710
GGG CTC TAT AAG TCT TCT GCT GGG AAT GCT CCC TGC TCA CCA TGC CCT 1758
GCC CGC AGT CAC GCT CCC AAC CCA GCA GCC CCC GTT TGC CCC TGC CTG 1806
GAG GGC TTC TAC CGG GCC AGT TCC GAC CCA CCA GAG GCC CCC TGC ACT 1854
GGT CCT CCA TCG GCT CCC CAG GAG CTT TGG TTT GAG GTG CAA GGC TCA 1902
GCA CTC ATG CTA CAC TGG CGC CTG CCT CGG GAG CTG GGG GGT CGA GGG 1950
GAC CTG CTC TTC AAT GTC GTG TGC AAG GAG TGT GAA GGC CGC CAG GAA 1998
CCT GCC AGC GGT GGT GGG GGC ACT TGT CAC CGC TGC AGG GAT GAG GTC 2046
CAC TTC GAC CCT CGC CAG AGA GGC CTG ACT GAG AGC CGA GTG TTA GTG 2094
GGG GGA CTC CGG GCA CAC GTA CCC TAC ATC TTA GAG GTG CAG GCT GTT 2142
AAT GGG GTG TCT GAG CTC AGC CCT GAC CCT CCT CAG GCT GCA GCC ATC 2190
AAT GTC AGC ACC AGC CAT GAA GTG CCC TCT GCT GTC CCT GTG GTG CAC 2238
CAG GTG AGC CGG GCA TCC AAC AGC ATC ACG GTG TCC TGG CCG CAG CCC 2286
GAC CAG ACC AAT GGG AAC ATC CTG GAC TAT CAG CTC CGC TAC TAT GAC 2334
CAG GCA GAA GAC GAA TCC CAC TCC TTC ACC CTG ACC AGC GAG ACC AAC 2382
ACT GCC ACC GTG ACA CAG CTG AGC CCT GGC CAC ATC TAT GGT TTC CAG 2430
GTG CGG GCC CGG ACT GCT GCC GGC CAC GGC CCC TAC GGG GGC AAA GTC 2478
TAT TTC CAG ACA CTT CCT CAA GGG GAG CTG TCT TCC CAG CTT CCG GAA 2526
AGA CTC TCC TTG GTG ATC GGC TCC ATC CTG GGG GCT TTG GCC TTC CTC 2574
CTG CTG GCA GCC ATC ACC GTG CTG GCG GTC GTC TTC CAG CGG AAG CGG 2622
CGT GGG ACT GGC TAC ACG GAG CAG CTG CAG CAA TAC AGC AGC CCA GGA 2670
CTC GGG GTG AAG TAT TAC ATC GAC CCC TCC ACC TAC GAG GAC CCC TGT 2718
CAG GCC ATC CGA GAA CTT GCC CGG GAA GTC GAT CCT GCT TAT ATC AAG 2766
ATT GAG GAG GTC ATT GGG ACA GGC TCT TTT GGA GAA GTG CGC CAG GGC 2814
CGC CTG CAG CCA CGG GGA CGG AGG GAG CAG ACT GTG GCC ATC CAG GCC 2862
CTG TGG GCC GGG GGC GCC GAA AGC CTG CAG ATG ACC TTC CTG GGC CGG 2910
GCC GCA GTG CTG GGT CAG TTC CAG CAC CCC AAC ATC CTG CGG CTG GAG 2958
GGC GTG GTC ACC AAG AGC CGA CCC CTC ATG GTG CTG ACG GAG TTC ATG 3006
GAG CTT GGC CCC CTG GAC AGC TTC CTC AGG CAG CGG GAG GGC CAG TTC 3054

AGC AGC CTG CAG CTG GTG GCC ATG CAG CGG GGA GTG GCT GCT GCC ATG 3102
CAG TAC CTG TCC AGC TTT GCC TTC GTC CAT CGC TCG CTG TCT GCC CAC 3150
AGC GTG CTG GTG AAT AGC CAC TTG GTG TGC AAG GTG GCC CGT CTT GGC 3198
CAC AGT CCT CAG GGC CCA AGT TGT TTG CTT CGC TGG GCA GCC CCA GAG 3246
GTC ATT GCA CAT GGA AAG CAT ACA ACA TCC AGT GAT GTC TGG AGC TTT 3294
GGG ATA CTC ATG TGG GAA GTG ATG AGT TAT GGA GAA CGG CCT TAC TGG 3342
GAC ATG AGT GAG CAG GAG GTA CTA AAT GCA ATA GAG CAG GAG TTC CGG 3390
CTG CCC CCG CCT CCA GGC TGT CCT CCT GGA TTA CAT CTA CTT ATG TTG 3438
GAC ACT TGG CAG AAG GAC CGT GCC CGG CGG CCT CAT TTT GAC CAG CTG 3486
GTG GCT GCA TTT GAC AAG ATG ATC CGC AAG CCA GAT ACC CTG CAG GCT 3534
GGC GGG GAC CCA GGG GAA AGG CCT TCC CAG GCC CTT CTG ACC CCT GTG 3582
GCC CTG GAC TTT CCT TGT CTG GAC TCA CCC CAG GCC TGG CTT TCA GCC 3630
ATT GGA CTG GAG TGC TAC CAG GAC AAC TTC TCC AAG TTT GGC CTC TGT 3678
ACC TTC AGT GAT GTG GCT CAG CTC AGC CTA GAA GAC CTG CCT GCC CTG 3726
GGC ATC ACC CTG GCT GGC CAC CAG AAG AAG CTG CTG CAC CAC ATC CAG 3774
CTC CTT CAG CAA CAC CTG AGG CAG CAG GGC TCA GTG GAG GTC 3816
TGAGAATGAC GATACCCGTG ACTCAGCCCT GGACACTGGT CCGAGAAGGG ACATGTGGGA 3876
CGTGAGCCGG GCTCCAACAG CCTCTGTGAG AGATGCCCCA CACCAAACCC AACCCTCCCG 3936
ATGGCTGCAT TCCCTGGTCC TCCGCCTCTC CACCAGCCCC CTCCTCATTA AAGGGAAAGA 3996
AGGGAATTTG CAAAAAAAAA AAAAAA 4022

SEQ ID NO : 3
Length : 24 nucleic acids
Type : nucleic acid
Strandedness : single
Topology : linear
Kind : synthesized DNA
TCAGGCCATC CGAGAACTTG CCCG

SEQ ID NO : 4
Length : 25 nucleic acids
Type : nucleic acid
Strandedness : single
Topology : linear
Kind : synthesized DNA
CATGTCCCAG TAAGGCCGTT CTCCA

### Brief Description of Drawings

Figure 1 is a chart of the structure of HEP receptor cDNA clone and a graph showing the result of hydrophobicity analysis.
Figure 2 shows the deduced amino acid sequence of HEP receptor aligned with amino acid sequences of Cek9 and Elk.
Figure 3 is a photograph instead of a drawing which shows the result of Northern blot analysis for HEP receptor gene.
Figure 4 is a photograph instead of a drawing which shows the result of Northern blot analysis for HEP receptor gene.
Figure 5 is a photograph instead of a drawing which shows the result of Immunoblot analysis by using an anti-HEP serum for wild type CHO-K1 cells (CHO-K1), transfectants of CHO-K1 cells into which a full length HEP receptor cDNA was introduced by an expression vector (CHO-HEP) and normal human brain (Brain#1, Brain#2).
Figure 6 A is a photograph instead of a drawing which shows the result of Immunoblot analysis by using an anti-HA monoclonal antibody for wild type CHO-K1 cells (CHO-K1), transfectants of CHO-K1 cells into which an FMS/HEP chimeric receptor cDNA and an FMS/HTK chimeric receptor cDNA were introduced by expression vectors (FMS/HEP and FMS/HTK). This photograph also shows the result of Immunoblot analysis by using an anti-FMS serum for wild type CHO-K1 cells and transfectants of CHO-K1 cells into which an FMS receptor cDNA was introduced by an expression vector (FMS).
Figure 6 B is a photograph instead of a drawing which shows the result of in vitro kinase assay for the FMS/HEP receptor protein, the FMS/HTK receptor protein and the FMS receptor protein.

## Claims

1. An Eph family receptor type tyrosine kinase-like protein in which an invariant lysine residue in ATP-binding site and an invariant aspartic acid residue in transphosphorylation site of Eph family receptor type tyrosine kinase are substituted with a glutamine residue and a serine residue, respectively.

2. The Eph family receptor type tyrosine kinase-like protein as claimed in claim 1, in which the invariant lysine residue in ATP-binding site, the invariant aspartic acid residue in transphosphorylation site, an invariant glutamic acid residue in kinase subdomain III and an invariant aspartic acid residue in kinase subdomain VI of Eph family receptor type tyrosine kinase are substituted with the glutamine residue, the serine residue, an arginine residue and a serine residue, respectively, and a triplet of Asp-Phe-Gly in kinase subdomain VII is substituted with a triplet of Arg-Leu-Gly.

3. The Eph family receptor type tyrosine kinase-like protein as claimed in claim 1, which contains an amino acid sequence from 17th Leu to 1,006th Val of that shown in SEQ ID No.:1.

4. The Eph family receptor type tyrosine kinase-like protein as claimed in claim 1, which contains an amino acid sequence from first Met to 1,006th Val of that shown in SEQ ID No.:1.

5. A DNA molecule which contains a nucleic acid sequence encoding the protein as claimed in any one of claims 1 to 4.

6. The DNA molecule as claimed in claim 5, which contains a nucleic acid sequence from 799th A to 3,816th C of that shown in SEQ ID No.:2.

7. An antibody which binds to the protein as claimed in any one of claims 1 to 4.
